# EUROPEAN PATENT SPECIFICATION

(11) **EP 2 963 890 B1**
(45) Date of publication and mention of the grant of the patent: **05.06.2019**
(21) Application number: 14290195.8
(22) Date of filing: 02.07.2014
(51) Int. Cl.: H04L 29/08, G06Q 50/22

(54) **GROUP COMMUNICATION APPARATUS**
GRUPPENKOMMUNIKATIONSVORRICHTUNG
APPAREIL DE COMMUNICATION DE GROUPE

(43) Date of publication of application: 06.01.2016
(73) Proprietor: Doro AB, 211 20 Malmö (SE)
(72) Inventor: Cullin, Peter, 245 44 Staffanstorp (SE); Arnaud, Jérôme, 92190 Meudon (FR); Criou, Acher, 92200 Neuilly-sur-Seine (FR); Palmqvist, Fredrik, 217 46 Malmö (SE); Kay, David, 211 28 Malmö (SE); Corbin, Xavier, 78460 Chevreuse (FR); Jacobsson, Fredrik, 261 40 Landskrona (SE); Johansson, Peter, 247 47 Flyinge (SE); Ingvarsson, Alf, 275 36 Sjöbo (SE)
(74) Representative: Ström & Gulliksson AB

(56) References cited:
- WO-A1-2013/077853
- WO-A2-2007/060558
- WO-A2-2009/137590
- US-A1- 2001 032 244
- US-A1- 2004 205 439
- US-A1- 2005 283 642
- US-A1- 2010 123 587
- US-A1- 2013 214 925
- US-A1- 2014 097 961

## Description

This application relates to a communications apparatus, a method, a mobile communications terminal, a system and a computer-readable storage medium for improved communication, and in particular to a communications apparatus, a method, a mobile communications terminal, a system and a computer-readable storage medium for addressing of messages to recipients.

### BACKGROUND

In today's society many different communications systems exist. Such communications systems may be rather advanced and comprise a vast multitude of functions and possible messages and requests to be sent. Also, there may be a plurality of receivers for the various types of messages and it may sometimes be unclear what action or function should be resolved by whom and to whom a message should be sent.

This can be confusing to many users and also difficult to overlook. It may cause a delay when sending a request if it is not readily known to whom a request should be sent and a recipient has to be searched for.

Also, if different users have clearance to or needs for different data, a plurality of requests may have to be sent if it is to be sent to different users having different clearances.

There is thus a need for a communications system wherein the selection of a recipient is simplified.

US 2010/123587 A1 presents a context of selective notifications to different groups of recipients according to their social/professional relationship with the message sender, however it does not disclose any selective retransmission of the confirmations or responses received for the notifications.

### SUMMARY

It is an object of the teachings of this application to overcome the problems listed above by providing a communication apparatus as defined in claim 1, a method as defined in claim 7 and a computer-readable storage medium as defined in claim 13. Claim 8 further defines a system comprising the communication apparatus of claim 1 and further detailed embodiments are defined by the other dependent claims.

The teachings herein find use in telecare systems but also in other communications systems.

Other features and advantages of the disclosed embodiments will appear from the following detailed disclosure, from the attached dependent claims as well as from the drawings.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of the element, device, component, means, step, etc., unless explicitly stated otherwise. The steps of any method disclosed herein do not have to be performed in the exact order disclosed, unless explicitly stated.

### BRIEF DESCRIPTION OF DRAWINGS

The teachings herein will be described in further detail under reference to the accompanying drawings in which:
Figure 1 shows a schematic view of a mobile communications terminal according to one embodiment of the teachings of this application;
Figure 2 shows a schematic view of the general structure of a mobile communications terminal according to one embodiment of the teachings of this application;
Figure 3 is a schematic view of a server according to the teachings herein;
Figure 4 is a schematic view of the components of a server according to the teachings herein;
Figure 5 show a schematic view of a telecommunications network according to one embodiment of the teachings of this application;
Figure 6 is a schematic general view of a communication system according to one embodiment of the teachings herein;
Figure 7 shows a schematic view of a computer-readable medium according to one embodiment of the teachings herein.
Figure 8 shows a schematic view of such a hierarchical arrangement of a communication system according to one embodiment of the teachings herein;
Figure 9 shows a general flowchart for a method of selecting a recipient based on a recipient layer model according to one embodiment of the teachings herein;
Figure 10 shows a schematic view of how data levels are associated with or cleared for different recipient layers according to one embodiment of the teachings herein;
Figure 11 shows a schematic view of such a hierarchical arrangement according to one embodiment of the teachings herein;
Figure 12 shows how a method according to herein is expanded with the optional filtering based on data according to one embodiment of the teachings herein;
Figure 13 shows a schematic view of a transparent recipient layer architecture according to one embodiment of the teachings herein;
Figure 14 shows a schematic view of the functionality of a message handler according to one embodiment of the teachings herein;
Figure 15 shows a schematic view of how a message handler directs and filters different types of messages according to one embodiment of the teachings herein;
Figure 16 shows a schematic view of a recipient layer hierarchy wherein confirmation that a communication request has been received or accepted and/or possibly carried out is transmitted from a recipient according to one embodiment of the teachings herein;
Figure 17 shows a flow chart of how a general method according to herein is expanded by the added functionality of sending confirmations according to one embodiment of the teachings herein;
Figure 18 shows a flow chart of how a general method according to herein is expanded by the added functionality of resending confirmations when no confirmations are received according to one embodiment of the teachings herein;
Figure 19 shows a schematic view of a recipient hierarchy and how a message/communication request may be deflected or forwarded according to one embodiment of the teachings herein;
Figure 20 shows a flow chart of how the general method according to herein is expanded by the added functionality of forwarding or deflecting a message or communication request according to one embodiment of the teachings herein;
Figure 21 shows a flow chart of a general method according to one embodiment of the teachings herein;
Figure 22 shows a flow chart of how a general method according to herein is expanded by the added functionality of selecting a recipient in a recipient layer based on the distance to the sender according to one embodiment of the teachings herein;
Figure 23 shows a flow chart of a general method according to one embodiment of the teachings herein;
Figure 24 shows a schematic overview of how a hierarchical arrangement may be used for a telecare communication system according to one embodiment of the teachings herein;
Figure 25 shows a schematic overview of how a hierarchical arrangement such may be used for forwarding a request according to one embodiment of the teachings herein;
Figure 26 shows a schematic overview of a telecare communication system hierarchy including an alarm center according to one embodiment of the teachings herein;
Figure 27 shows a flowchart of how a general method according to herein may be expanded to include an alarm functionality according to one embodiment of the teachings herein;
Figure 28 shows a schematic overview of a telecare communication system hierarchy including a safety timer functionality according to one embodiment of the teachings herein;
Figure 29 shows a flowchart of how a general method according to herein may be expanded to include this safety timer functionality according to one embodiment of the teachings herein;
Figure 30 shows a schematic overview of a telecare communication system hierarchy including an are you ok functionality according to one embodiment of the teachings herein;
Figure 31 shows a flowchart of how a general method according to herein may be expanded to include such an are you ok functionality according to one embodiment of the teachings herein;
Figure 32 shows a schematic overview of a telecare communication system hierarchy including a monitoring functionality according to one embodiment of the teachings herein;
Figure 33 shows a flowchart of a monitoring functionality according to one embodiment of the teachings herein;
Figure 34 shows an overview of a mobile communication terminal according to one embodiment of the teachings herein; and
Figure 35 shows an overview of a mobile communication terminal according to one embodiment of the teachings herein.

### DETAILED DESCRIPTION

The disclosed embodiments will now be described more fully hereinafter with reference to the accompanying drawings, in which certain embodiments of the invention are shown. This invention may, however, be embodied in many different forms and should not be construed as limited to the embodiments set forth herein; rather, these embodiments are provided by way of example so that this disclosure will be thorough and complete, and will fully convey the scope of the invention to those skilled in the art. Like numbers refer to like elements throughout.

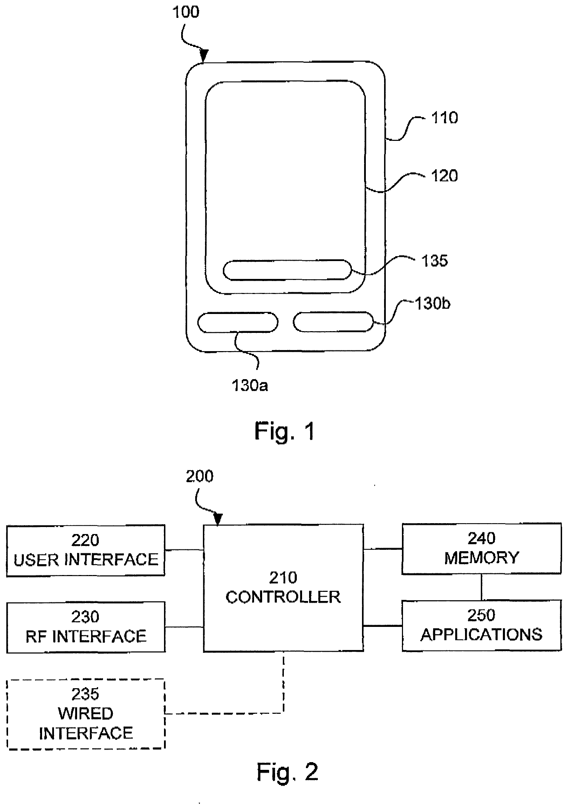

Figure 1 shows a schematic overview of a mobile communications terminal 100 adapted according to the teachings herein. In the embodiment shown the mobile communications terminal is a mobile phone 100. In other embodiments the mobile communications terminal 100 is computer tablet, a laptop computer, a personal digital assistant, a media player, a location finding device or any hand-held device capable of communicating with other devices. The mobile phone 100 comprises a housing 110 in which a display 120 is arranged. In one embodiment the display 120 is a touch display. In other embodiments the display 120 is a non-touch display. Furthermore, the mobile phone 100 comprises two keys 130a, 130b. In this embodiment there are two keys 130, but any number of keys, including none, is possible and depends on the design of the mobile phone 100. In one embodiment the mobile phone 100 is configured to display and operate a virtual key 135 on the touch display 120. It should be noted that the number of virtual keys 135 are dependent on the design of the mobile phone 100 and an application that is executed on the mobile phone 100. In one embodiment the communications terminal 100 comprises an ITU-T keypad or a QWERTY (or equivalent) keypad in addition to or as an alternative to a touch-sensitive display. In an embodiment where the keypad is an alternative to a touch-sensitive display, the display 120 is a non-touch-sensitive display.

Figure 2 shows a schematic view of the general structure of a mobile communications terminal 200 (100) according to figure 1. The mobile communications terminal 200 comprises a controller 210 which is responsible for the overall operation of the mobile communications terminal 200 and is preferably implemented by any commercially available CPU ("Central Processing Unit"), DSP ("Digital Signal Processor") or any other electronic programmable logic device. The controller 210 is implemented using instructions that enable hardware functionality, for example, by using computer program instructions executable in a general-purpose or special-purpose processor that may be stored on a computer readable storage medium (disk, memory etc) 240 to be executed by such a processor. The controller 210 is configured to read instructions from the memory 240 and execute these instructions to control the operation of the mobile communications terminal 100. The memory 240 may be implemented using any commonly known technology for computer-readable memories such as ROM, RAM, SRAM, DRAM, CMOS, FLASH, DDR, EEPROM memory, flash memory, hard drive, optical storage or any combination thereof. The memory 240 is used for various purposes by the controller 210, one of them being for storing application data and various software modules in the mobile terminal. The software modules include a real-time operating system, an application handler as well as various applications 250. The applications 250 are sets of instructions that when executed by the controller 210 control the operation of the mobile communications terminal 100. The applications 250 can include a messaging application for short messaging service (SMS), multimedia messaging service (MMS) and electronic mail, a media player application, as well as various other applications 250, such as applications for voice calling, video calling, web browsing, document reading and/or document editing, an instant messaging application, a phonebook application, a calendar application, a control panel application, one or more video games, a notepad application, location finding applications, et.c.. A message handler may be implemented and stored in the memory 240 as an application 250 for handling messages sent from and received at the mobile communication terminal 200.

The mobile communications terminal 200 further comprises drivers for a user interface 220, which in the mobile communications terminal 100 of figure 1 is comprised of the display 120, the keys 130, 135, a microphone and a loudspeaker. The user interface (UI) drivers 220 also includes one or more hardware controllers, which together with the UI drivers cooperate with the display 120, keypad 130, as well as various other I/O devices such as microphone, loudspeaker, vibrator, ringtone generator, LED indicator, etc. As is commonly known, the user may operate the mobile terminal through the man-machine interface thus formed.

The mobile communications terminal 200 further comprises a radio frequency interface 230, which is adapted to allow the mobile communications terminal to communicate with other communication terminals in a radio frequency band through the use of different radio frequency technologies. Examples of such technologies are W-CDMA, GSM, UTRAN, LTE and NMT to name a few. The controller 210 is configured to operably execute the applications 250, such as the voice call and message handling applications, through the RF interface 230 and software stored in the memory 240, which software includes various modules, protocol stacks, drivers, etc. to provide communication services (such as transport, network and connectivity) for the RF interface 230, and optionally a Bluetooth interface and/or an IrDA interface for local connectivity. The RF interface 230 comprises an internal or external antenna as well as appropriate radio circuitry for establishing and maintaining a wireless link to a base station. As is well known to a person skilled in the art, the radio circuitry comprises a series of analogue and digital electronic components, together forming a radio receiver and transmitter. These components include, i.e., band pass filters, amplifiers, mixers, local oscillators, low pass filters, AD/DA converters, etc.

In one embodiment the mobile communications terminal 200 further comprises a wired interface 235 (indicated with a dashed line in figure 2 as this is an optional feature), which is adapted to allow the terminal to communicate with other devices through the use of different network technologies. Examples of such technologies are USB (Universal Serial Bus), Ethernet, Local Area Network, and TCP/IP (Transport Control Protocol/Internet Protocol) to name a few.

The RF interface 230 and the wired interface 235 are examples of device communication interfaces that enable communication between the mobile communications terminal 200 and another device.

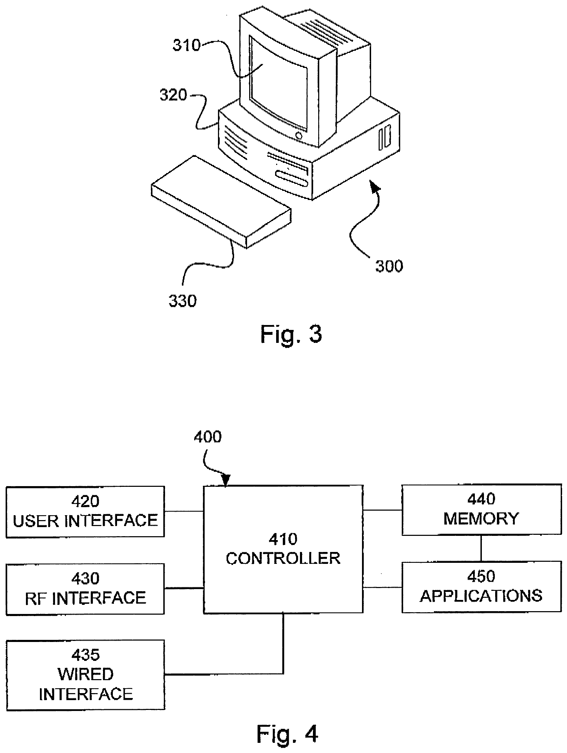

Figure 3 shows a server 300 according to an embodiment herein. In one embodiment the server 300 is configured for network communication, either wireless or wired. In one embodiment the server 300 is configured for network communication, both wireless and wired. Examples of such a server 300 are: a personal computer, desktop or laptop computer, an internet tablet computer, a mobile telephone, a smart phone, a personal digital assistant and a work station.

The server 300 will hereafter be exemplified and described as being a computer 300. The computer or terminal 300 comprises a housing 320 and may comprise a display 310. The housing comprises a controller or CPU (not shown) and one or more computer-readable storage mediums (not shown), such as storage units and internal memory. Examples of storage units are disk drives or hard drives. The server 300 further comprises at least one data port. Data ports can be wired and/or wireless. Examples of data ports are USB (Universal Serial Bus) ports, Ethernet ports or Wi-Fi (according to IEEE standard 802.11) ports. Data ports are configured to enable a terminal 300 to connect with other terminals or servers.

The terminal 300 may further comprise at least one input unit such as a keyboard 330. Other examples of input units are computer mouse, touch pads, touch screens or joysticks to name a few.

In one embodiment the server 300 is a network server. In an alternative embodiment the server 300 is implemented as a software module (250) to be executed by the mobile communication terminal 100, 200 of figures 1 and 2. A message handler may be implemented as part of the software module for handling messages sent from and received at the mobile communication terminal 200.

As the message handler may be implemented centrally in a server, that in turn may be implemented in a mobile communication terminal or a server, or locally in a mobile communication terminal, it will be understood herein that the message handler is implemented in a communication apparatus that in turn may be implemented in a mobile communication terminal, as a server module in a mobile communication terminal, in a server, such as a computer, or a combination thereof.

Figure 4 shows a schematic view of the general structure of a server 400 (300) according to figure 3. The server is a computer terminal 400 in one embodiment. A computer terminal 400 call be used as a server or a client and also both as a server and a client as would be appreciated by a skilled person. With reference to figure 4 the server will be described as being a personal computer or terminal 400, but it should be noted that a server may have the same or similar components. Typically for a server only one user interface is provided for an arrangement of computers or servers. The terminal 400 comprises a controller 410 which is responsible for the overall operation of the terminal 400 and is implemented by any commercially available CPU ("Central Processing Unit"), DSP ("Digital Signal Processor") or any other electronic programmable logic device. The controller 410 is implemented using instructions that enable hardware functionality, for example, by using executable computer program instructions in a general-purpose or special-purpose processor that may be stored on a computer readable storage medium (disk, memory etc) 440 to be executed by such a processor. The controller 410 is configured to read instructions from the memory 440 and execute these instructions to control the operation of the terminal 400. The memory 440 is implemented using any commonly known technology for computer-readable memories such as ROM, RAM, SRAM, DRAM, CMOS, FLASH, DDR, EEPROM memory, flash memory, hard drive, optical storage or any combination thereof. The terminal 400 further comprises one or more applications 450. The applications are sets of instructions that when executed by the controller 410 control the operation of the terminal 400. The memory 440 is used for various purposes by the controller 410, one of them being for storing application data and program instructions 450 for various software modules in the terminal 400. The software modules include a real-time operating system, drivers for a man-machine interface 420, an application handler as well as various applications 450. The applications 450 can include a messaging application such as electronic mail, a browsing application, a media player application, as well as various other applications 450, such as applications for voice calling, video calling, document reading and/or document editing, an instant messaging application, a calendar application, a control panel application, one or more video games, a notepad application, etc.

A message handler may be implemented and stored in the memory 440 as an application 450 for handling messages sent from and received at the mobile communication terminal 200.

The terminal 400 may further comprise drivers for a user interface 420 which in the terminal 300 of figure of 3, is comprised of the display 310 and the keypad 330. The user interface (UI) drivers 420 also includes one or more hardware controllers, which together with the UI drivers cooperate with the display 310, keypad 330, as well as various other I/O devices such as sound system, LED indicator, etc. As is commonly known, the user may operate the terminal 400 through the man-machine interface thus formed.

The terminal 400 further comprises a radio frequency interface 430, which is adapted to allow the terminal to communicate with other devices through a radio frequency band through the use of different radio frequency technologies. Examples of such technologies are WIFI, Bluetooth®, W-CDMA, GSM, UTRAN, LTE, and NMT to name a few.

The terminal 400 further comprises a wired interface 435, which is adapted to allow the terminal to communicate with other devices through the use of different network technologies. Examples of such technologies are USB, Ethernet, and Local Area Network, TCP/IP (Transport Control Protocol/Internet Protocol) to name a few.

The controller 410 is configured to operably execute applications 450 such as the web browsing or email application through the RF interface 430 and/or the wired interface 435 using software stored in the memory 440 which software includes various modules, protocol stacks, drivers, etc. to provide communication services (such as transport, network and connectivity) for the RF interface 430 and the wired interface 435, and optionally a Bluetooth interface and/or an IrDA interface for local connectivity. The RF interface 430 comprises an internal or external antenna as well as appropriate radio circuitry for establishing and maintaining a wireless link to a base station. As is well known to a person skilled in the art, the radio circuitry comprises a series of analogue and digital electronic components, together forming a radio receiver and transmitter. These components include, i.e., band pass filters, amplifiers, mixers, local oscillators, low pass filters, AD/DA converters, etc. The RF interface 430, the wired interface 435, the Bluetooth interface and the IrDA interface are examples of device communication interfaces that enable communication between the terminal 400 and another device.

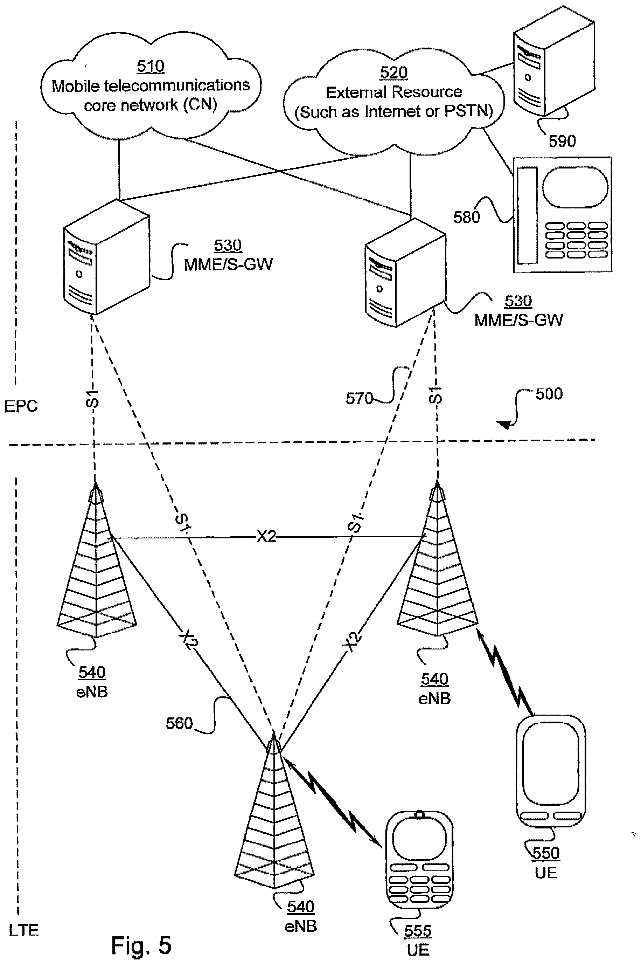

Figure 5 shows a schematic view of the general structure of a telecommunications system 500 according to the teachings herein. In the telecommunication system of figure 5, various telecommunications services such as cellular voice calls, www/wap browsing, cellular video calls, data calls, facsimile transmissions, music transmissions, still image transmissions, video transmissions, electronic message transmissions and electronic commerce may be performed between a mobile terminal 100, 300, 550 according to the disclosed embodiments and other communications terminals, such as another mobile terminal 555 or a stationary telephone 580. The mobile terminals 550, 555 are connected to a mobile telecommunications network 510 through Radio Frequency links via base stations 540.

The telecommunications system 500 comprises at least one server 530. A server 530 has a data storage and a controller that is implemented by any commercially available CPU ("Central Processing Unit"), DSP ("Digital Signal Processor") or any other electronic programmable logic device. In one embodiment such a server is a Mobility Management Entity (MME). In one embodiment such a server is a Gateway (GW). The servers 530 are configured to communicate with a mobile telecommunications core network (CN) 510 and/or an external resource 520 such as the internet or a Public Switched Telephone Network (PSTN). A PSTN 520 is configured to communicate with and establish communication between stationary or portable telephones 580. In one embodiment the external resource comprises or is configured to communicate with an external service provider 590. In one embodiment the servers 530 are configured to communicate with other communications terminals using a packet switched technology or protocol. In such an embodiment the servers 530 may make up an Evolved Packet Core (EPC) layer.

The servers 530 are configured to communicate with nodes, also referred to as base stations 540. In one embodiment the base station 540 is an evolved Node Base (eNB). A base station 540 is further configured to communicate with a server 530. In one embodiment the communication between a server 530 and a base station 540 is effected through a standard or protocol 570. In one embodiment the protocol is S1. A base station 540 is configured to communicate with another base station 540. In one embodiment the communication between a base station 540 and another base station 540 is effected through a standard or protocol 560- In one embodiment the protocol 560 is X2. A base station 540 is further configured to handle or service a cell 580. In one embodiment the at least one base stations 540 make up a Long Term Evolution (LTE) layer. In one embodiment the at least one base stations 540 make up an LTE Advanced layer.

In one embodiment the base station 540 is configured to communicate with a mobile communications terminal 550 (100, 300) through a wireless radio frequency protocol.

In one embodiment the telecommunications system 500 is an Evolved Packet System (EPS) network. In one embodiment the telecommunications system is a system based on the 3GPP (3^{rd} Generation Partnership Project) standard. In one embodiment the telecommunications system is a system based on the UMTS (Universal Mobile Telecommunications System) standard. In one embodiment the telecommunications system is a system based on a telecommunications standard such as GSM, D-AMPS, CDMA2000, FOMA or TD-SCDMA.

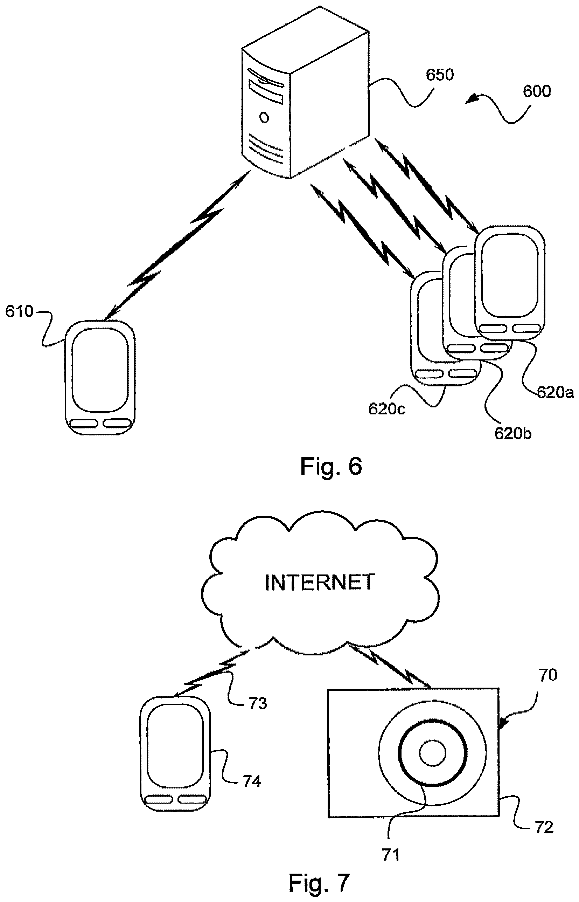

Figure 6 6 shows a schematic overview of a communications system 600 according to an embodiment herein. A mobile communication terminal 610, 620, such as the mobile communication terminals 100, 200 of figures 1 and/or 2 is connected to a communications network (indicated by the arrows), for example the internet or a telecommunications network as per figure 5, for enabling communication between the various mobile communication terminals. In figure 6 there are four mobile communication terminals 610 and 620a-c. The communications system 600 may further comprise at least one server 650. The server is in one embodiment a server 300, 400 according to figures 3 or 4 and in one embodiment the server is a software module 250 implemented in a mobile communication terminal 100, 200 according to figures 1 or 2. In figure 6 only one server 650 is shown, but it should be noted that any number of servers 650 may be implemented in a communications system 600. Generally a server is a physical computer (a hardware system) or a software module dedicated to running one or more services (as a host), to serve the needs of users of the mobile communication terminals 610, 620 in the system 600.

In one embodiment the communications system 600 and/or the communications network of figure 6 forms part of the External Resources 520 of figure 5. And, in one embodiment, the communications network 500 of figure 5 forms part of the computer system 600 of figure 6. In these embodiments the mobile communication terminals 610, 620 of figure 6 correspond to mobile communication terminals 550 and 555 of figure 5.

As would be apparent to a skilled reader, a computer-enabled communications network, such as the Internet or a telecommunications network, provides many possibilities and variations of how to connect two terminals, and the embodiments disclosed herein are for purely exemplary purposes and should not be construed to be limiting-

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other devices. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

Figure 7 shows a schematic view of a computer-readable storage medium, such as a computer-readable storage medium, as described in the above. The computer-readable medium 70 is in this embodiment a data disc 70. In one embodiment the data disc 70 is a magnetic data storage disc. The data disc 70 is configured to carry instructions 71 that when loaded into a controller, such as a processor, executes a method or procedure according to the embodiments disclosed above. The data disc 70 is arranged to be connected to or within and read by a reading device 72, for loading the instructions into the controller. One such example of a reading device 72 in combination with one (or several) data disc(s) 70 is a hard drive. It should be noted that the computer-readable medium can also be other mediums such as compact discs, digital video discs, flash memories or other memory technologies commonly used.

The instructions 71 may also be downloaded to a computer data reading device 74, such as a mobile communications terminal 74 or other device capable of reading computer coded data on a computer-readable medium, by comprising the instructions 71 in a computer-readable signal 73 which is transmitted via a wireless (or wired) interface (for example via the Internet) to the computer data reading device 74 for loading the instructions 71 into a controller. In such an embodiment the computer-readable signal 73 is one type of a computer-readable medium 70.

The instructions may be stored in a memory (not shown explicitly in figure 7, but referenced 240 in figure 2) of the mobile communications terminal 74.

References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

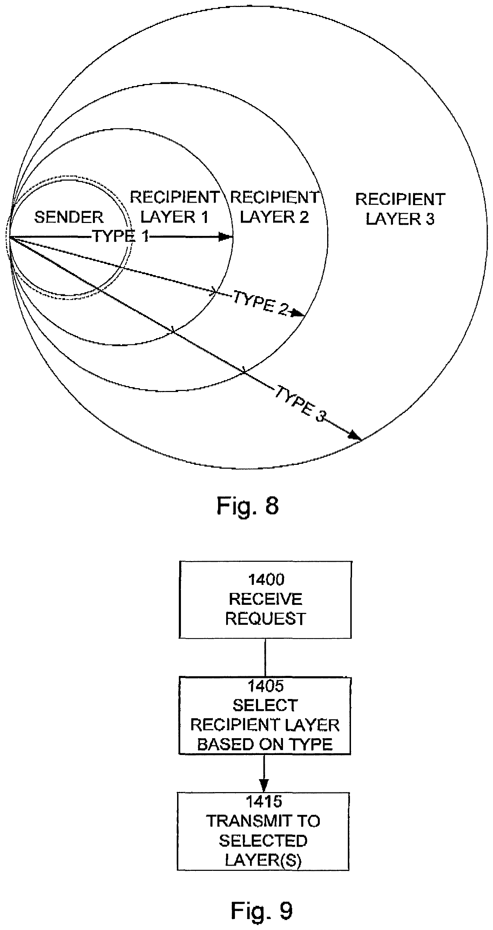

To enable a smoother and easier communication between users of different mobile communication terminals, where one user may be a user that is cared for by the other users, the communication system 600 of figure 6 is arranged in a hierarchical structure where mobile communication terminals are arranged in different layers. Primarily there are two different types of layers, a sending layer and one or more receiving layers. Figure 8 shows a schematic view of such a hierarchical arrangement of a communication system. The layering may be administered centrally by a server (possibly implemented as a software module in one mobile communication terminal) or distributed among the various devices in the communication system 600. In the example of figure 8, there is shown one sender layer SENDER) and three recipient layers RECIPIENT LAYER 1, RECIPIENT LAYER2, RECIPIENT LAYER3.

As in any communication system, the various devices are configured to communicate with one another by sending messages. To enable for a smooth and efficient message sending allowing a user or SENDER to select a message and its recipients quickly and without technical knowledge or much knowledge of communication systems, at least a portion of the messages that may be sent in the communication system is arranged with a message type. In figure 8, there are three different types being shown, TYPE 1, TYPE 2, and TYPE 3. The layering of the recipients is associated with different types of messages so that a message handler (not shown in figure 8, but will be discussed in detail in the below) may select recipients for a message based on the type of message. As is indicated by the dashed circle around the sender layer SENDER in figure 8, the sender user may not be aware of who the users are in the recipient layers RECIPIENT LAYER X. A user sending a message would then not need to know, select or concern himself with who the actual recipient of the message is which simplifies generation and sending of the message as no recipient needs to explicitly selected or indicated by the sender SENDER.

The recipient layers (RECIPIENT LAYER X) may be generated based on a phonebook or other contact list stored in the sender mobile communication terminal (referenced 610 in figure 6) or in a recipient mobile communication terminal (referenced 620 in figure 6). Alternatively or additionally, the recipient layers may be generated based on group listings in social media applications.

A recipient layer may comprise more than one user or recipient and as a recipient layer is selected based on a message type, all recipients in the selected recipient layer may be selected as recipients or a subset, possibly being only one recipient, may be selected as recipient.

The messages may be generated based on a pre-specified type. In this manner a user may simply select a message type from a number of predefined message types and a message is automatically generated and possibly also sent by the user issuing a single simple command such as pressing a virtual key.

Alternatively or additionally, the type of a message being or already generated may be determined based on the content of the message. This allows a user to generate a message without knowing the different available types by simply typing in or otherwise generating his message (for example by selecting keywords or following instructions in a message generating wizard or form) and the message is then sent to a recipient layer based on the information in the message-

Even though figure 8 only shows three different types of messages (TYPE 1, TYPE 2, TYPE 3, it should be noted that more than one type of message may be sent to the same recipient layer.

Message types TYPE X may be prioritized in such a manner that a higher prioritized message (TYPE 1 for example) may only be sent to high level recipient layers (RECIPIENT LAYER 1 for example), whereas a lower prioritized message (TYPE 1 for example) may be sent to the corresponding low level recipient layers (RECIPIENT LAYER 3 for example) as well as to higher recipient layers (RECIPIENT LAYERS 1 and 2 for example). This is indicated in figure 8 by the arrows for message types 2 and 3 having smaller arrows when intersecting the upper layers, layers 1 and 2.

It should be noted that more than one type may be associated with each or any recipient layer even though the figures only show three different message types.

In one embodiment the messages are communication requests and the types are communication request types. For handling communication requests it is beneficial to a user if he need not know or find out exactly to whom a communication request has to be sent to, especially as a user is often not concerned with who executes a communication request, only that the communication request is answered and executed. The sending user thus only have to focus on selecting a proper communication request and the communication system will handle the rest including selecting recipients and transmitting the communication request.

Figure 9 shows a general flowchart for a method of selecting a recipient based on the recipient layer model of figure 8. A communication request or other message is received 1400 by a message handler being implemented in a server (for centralized control) or a message handler application (250) (for distributed control). It should be noted that the steps may be performed in unison by one and the same apparatus, for example the server, or some actins may be executed by one apparatus, and other actions executed or performed by another apparatus. The same is true for all flow charts disclosed herein.

As the communication request has been received a recipient layer is selected 1405 based on the message type and the communication request is transmitted to the selected layer(s) 1415. As noted in the above, a message or communication request type may be associated with more than one layer.

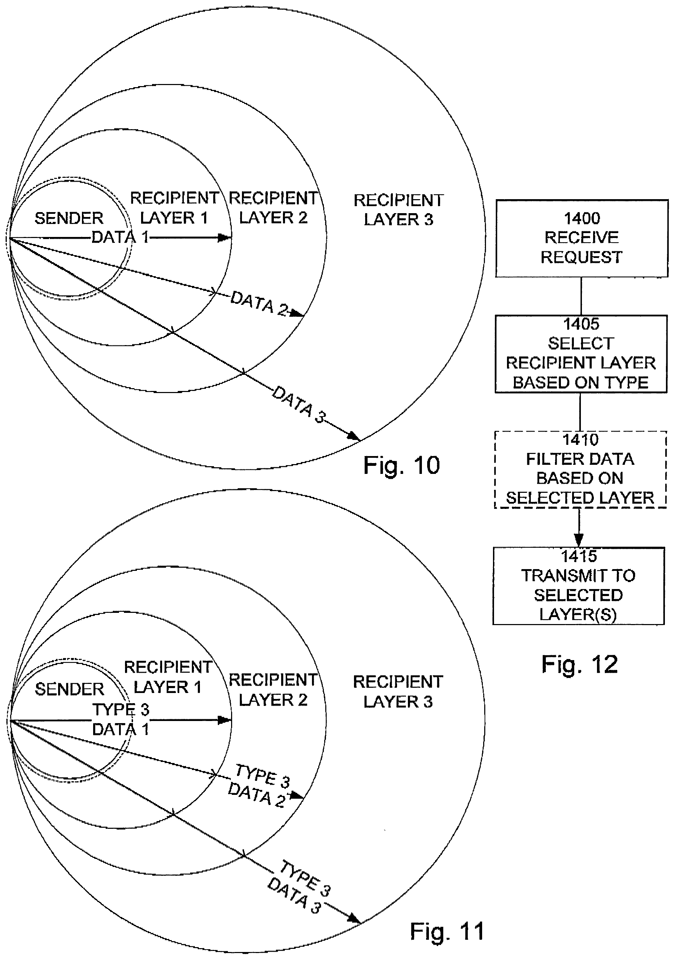

In one embodiment a message type may also be associated with a data structure possibly having or indicating different data levels and the different recipient layers may be cleared for different data levels. Figure 10 schematically shows how such data levels are associated with or cleared for the different recipient layers. For example, the highest recipient layer RECIPIENT LAYER 1 illustrated as the innermost layer in figure 10, is associated with a high data level DATA 1, the middle recipient layer RECIPIENT LAYER 2, is associated with a middle data level DATA 2, and the lowest recipient layer RECIPIENT LAYER. 3 illustrated as the outermost layer in figure 10, is associated with a low data level DATA 3. For the purpose of this application a higher layer will refer to a layer having a higher priority or clearance or a stronger association with the sender, and a lower layer will refer to a layer having a lower priority or clearance or a weaker if any association with the sender.

This enables data to be filtered and sorted based on the message type which enables a sender to not have to worry about or indicate what data is sent to which recipient

As one communication request type may be sent to more than one layer, it can be good to filter the data that is shown or transmitted to the different recipient layers to enable for a hierarchical data layering as well. The teachings herein thus provide for a manner in which a user may send a request to multiple recipients without having to know who the recipients are and what data to send to each recipient. Figure 11 shows a schematic view of such a hierarchical arrangement. In this example a message having a request type TYPE 3 is sent to all recipient layers, but the data being transmitted to the various recipient layers has been filtered based on the recipient layer which in turn is based on the request type, thereby implementing a filtering based on the type. In the example of figure 11a message is sent to RECIPIENT LAYER 1 with DATA 1, to RECIPIENT LAYER 2 with DATA 2 and to RECIPIENT LAYER 3 with DATA 3.

One example of different data levels is for a medical application, wherein the data levels are arranged in the following: [LAYER3: CRUCIAL MEDICINES; LAYER 2: ALL MEDICINES; LAYER 1: MEDICINES AND REASON/ILLNESS]. Another example of different data levels is for a confidential data application, wherein the data levels are arranged in the following: [LAYER 3: TIMELIMITS AND ACTIONS; LAYER 2: ALSO SUBJECT MATTER; LAYER 1: ALSO PROPOSED ACTION].

Figure 12 shows how the general flowchart of a method according to herein is expanded with the optional filtering based on data. The filtering is optional as is indicated by the dashed lines.

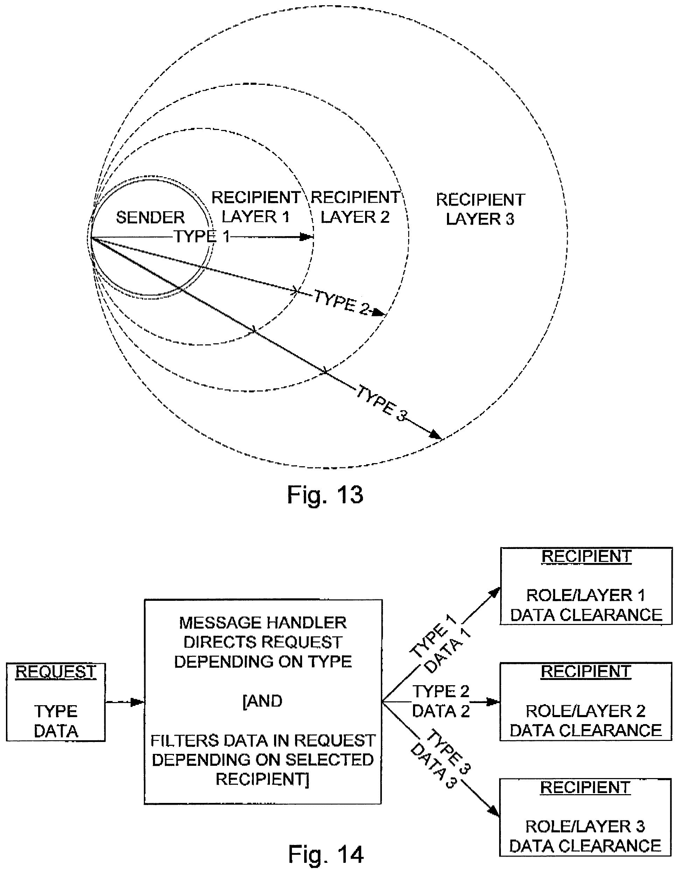

As has been noted in the above, the sender may not be aware of who is in the various recipient layers and in a similar manner the different recipients in the different recipient layers may not know who is in the other recipient layers. This simplifies the inter-recipient layer communication. The recipient layers may also be transparent with regards to the recipients in each recipient layer. Figure 13 shows a schematic view of a transparent recipient layer architecture.

A message handler has been discussed in the above to be able to receive a communication request and direct it according to its type. The message handler may be implemented centrally in a server (possibly as a software module) or distributed wherein different devices or apparatuses are configured or arranged to implement sub functionalities of the message handler.

Figure 14 shows a schematic view of the functionality of the message handler, wherein a REQUEST or other message is received, which communication request carries or may be determined to be of a type TYPE. Optionally the communication request also carries data DATA. The message handler selects a recipient layer (and thereby a recipient) based on the message type, possibly after having determined the message type, and optionally filters the data depending on the selected recipient and then directs or transmits the communication request accordingly. The message handler may be implemented to only direct the message/communication request and another module may then carry out the actual transmittal of the message/communication request or the message handler may also transmit the message as a recipient has been selected.

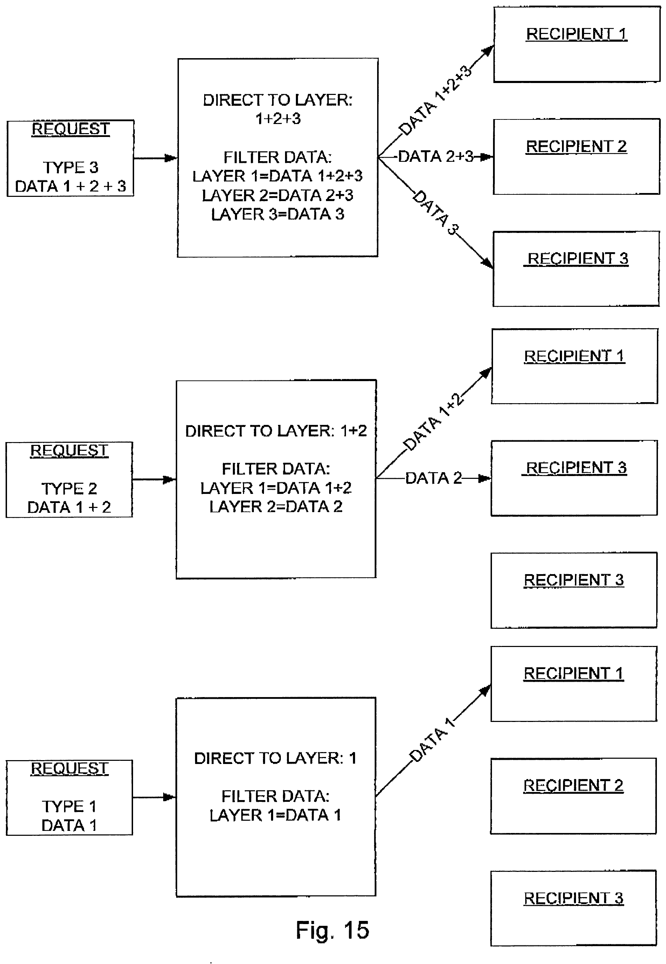

Figure 15 shows a schematic view of how the message handler directs and filters different types of messages. In the example of figure 15, three different communication requests are being handled, one of TYPE 1, carrying data for RECIPIENT LEVEL 1, one of TYPE 2, carrying data for RECIPIENT LEVEL 1 and RECIPIENT LEVEL 2 and one of TYPE 3, carrying data for RECIPIENT LEVEL 1, RECIPIENT LEVEL 2 and RECIPIENT LEVEL 3. The various communication requests are transmitted to the selected recipients based on the request type and carries the data associated with the corresponding recipient layers.

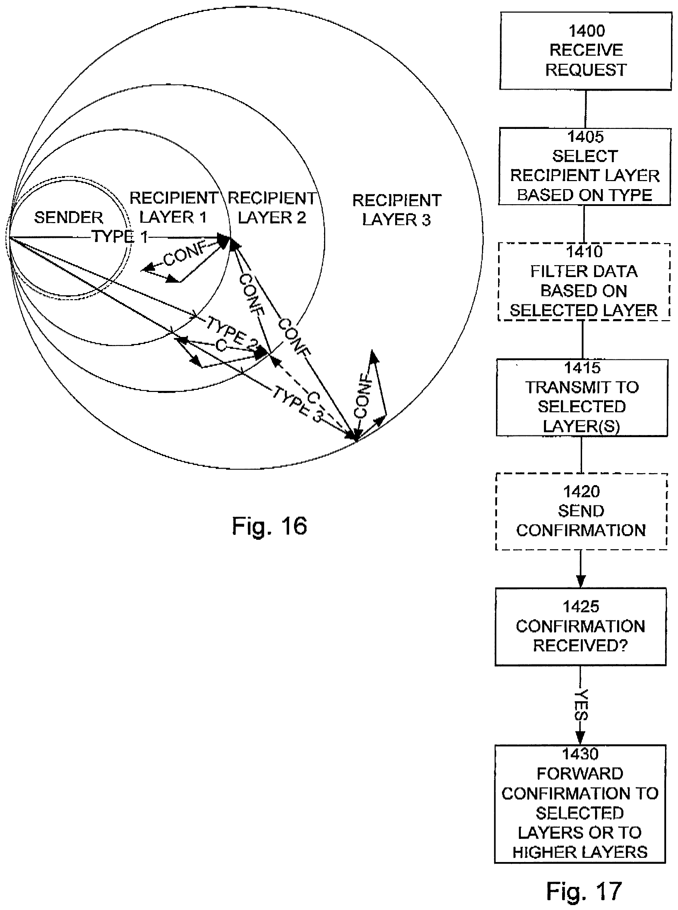

As a communication request or message may be sent to more than one recipient in each recipient layer there is a risk that the recipients do not know who should deal with a specific communication request or if the communication request has already been dealt with. Also, a recipient needs to be informed that a communication request actually has been handled so that the recipient need not worry about it anymore. Also, to prevent that a communication request is forgotten and to prevent such double work a manner to confirm a communication request is presented in figure 16 showing a schematic view of a recipient layer hierarchy wherein confirmation that a communication request has been received or accepted and/or possibly carried out is transmitted from a recipient. The confirmation (referenced CONF or C in figure 16) may be transmitted directly to the other recipients in the recipient layer, or to a message handler for forwarding to the other recipients. A confirmation may also be sent to other recipient layers as is indicated in figure 16.

Figure 17 shows a flow chart of how the general method according to herein is expanded by the added functionality of sending confirmations.

As a communication request has been transmitted the recipient sends a confirmation 1420. This transmission is indicated with dashed arrows as it may be executed by a different device than the one(s) executing the rest of the method. As a confirmation is received 1425 the confirmation may be forwarded 1430 to selected layers or to higher layers so that the higher layers are informed of the progress of a communication request. It should be noted that the forwarding 1430 may be included in the sending of the confirmation 1420 if a recipient sends his confirmation directly to other recipients. Combinations are also possible.

In one embodiment the highest recipient layer RECIPIENT LAYER 1, is informed of the status or progress of all communication requests by receiving (a copy of) every communication request and also receiving (a copy of) every confirmation.

In one embodiment the message handler is configured to resend a communication request if no confirmation is received. This may be done automatically or after prompting by a recipient in a higher recipient layer, for example in RECIPIENT LAYER 1. Such prompting is then received by the message handler and acted upon.

The message handler may alternatively be arranged to send out a reminder of the communication request. A reminder is beneficial in that it usually requires less bandwidth to be transmitted and also avoids resending possibly sensitive data.

Resending the communication request is beneficial in that a recipient will know what the communication request relates to - especially beneficial if the original communication request was simply not received.

Optionally a higher level recipient or the message handler can select a new recipient layer from a lower level if no confirmation has been received. This would ensure that a communication request is handled, since a larger recipient group is targeted.

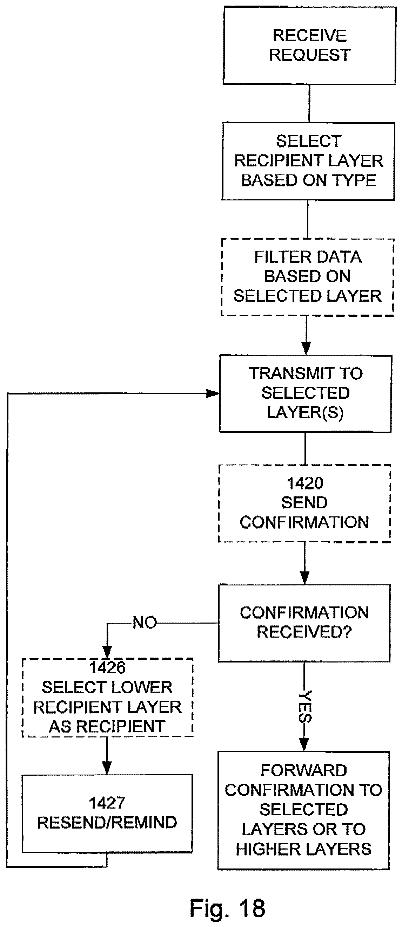

Figure 18 shows a flow chart of how the general method according to herein is expanded by the added functionality of resending confirmations when no confirmations are received. If no confirmation is received, a new recipient layer may optionally be selected 1426 (as is indicated by the dashed lines) and a reminder is transmitted or the communication request is retransmitted. Alternatively a reminder may be sent out to those recipients who have already received the communication request and the communication request is resent to those that have not received it yet.

To ensure that a communication request is handled a higher level recipient may choose to delegate or distribute a communication request by deflecting or forwarding a communication request to a lower recipient layer.

As the recipient forwards or deflects a communication request any data carried in the communication request may be filtered based on the recipient layer to be forwarded to, or the communication request is forwarded as it was received.

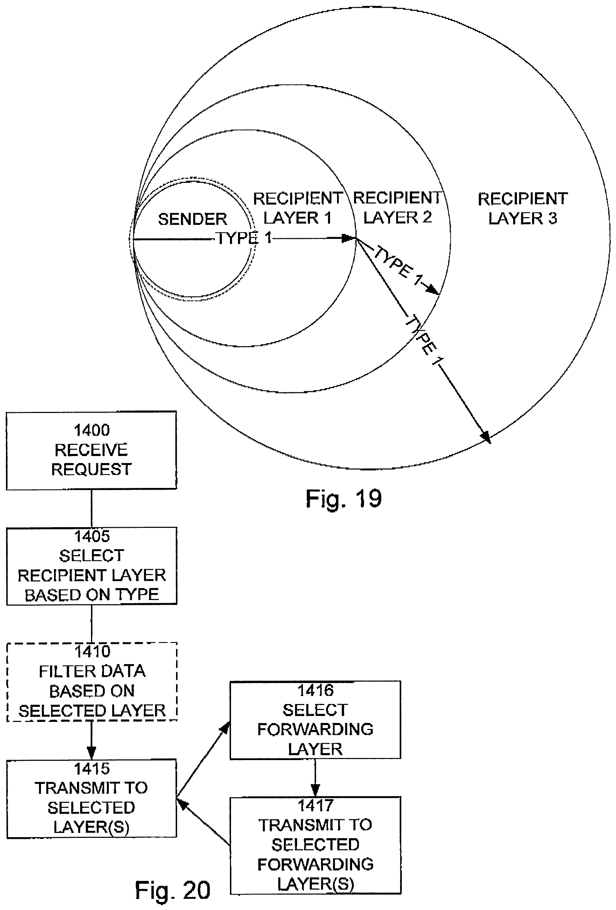

Figure 19 shows a schematic view of the recipient hierarchy and how a message/communication request may be deflected or forwarded.

The difference between a forwarded and a deflected message for the content of this application is that a deflected message or communication request will not be handled by the original receiver, whereas a forwarded communication request/message may be handled by any recipient By deflecting a message, the user thus delegates the communication request/message to someone else, whereas by forwarding it the recipient simply expands the number of recipients.

Figure 20 shows a flow chart of how the general method according to herein is expanded by the added functionality of forwarding or deflecting a message or communication request. As a communication request or message is to be forwarded or deflected a recipient layer is selected 1416. This may be selected automatically by a further association of a message type and a recipient layer. Alternatively, a recipient may indicate to which recipient layer the communication request or message should be forwarded/deflected. Then the message/communication request is forwarded or deflected 1417.

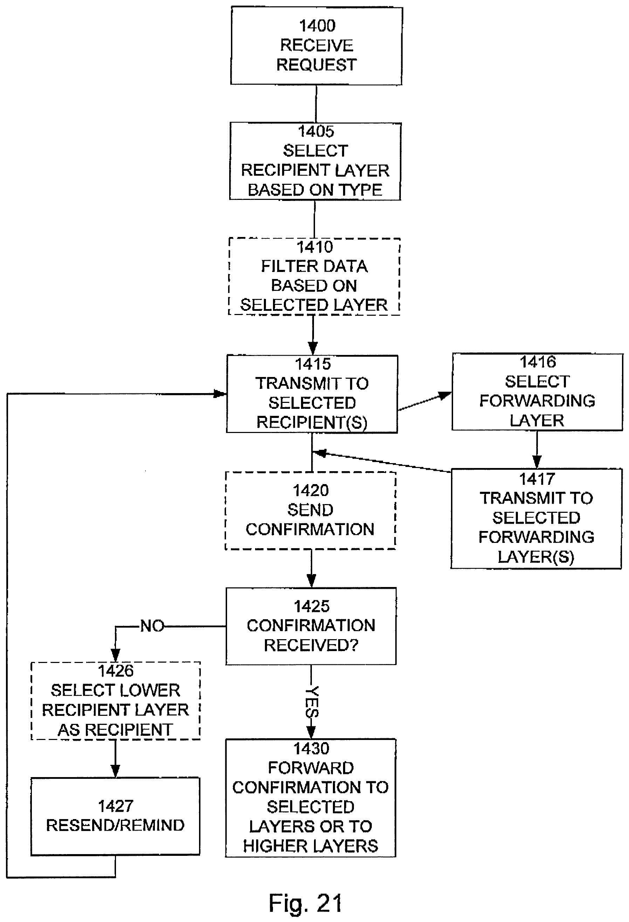

Figure 21 shows a flow chart of a general method according to herein encompassing all features disclosed in the above.

To ensure that a communication request is not sent to a recipient in vain, the distance to the recipient may be taken into account when selecting a recipient layer. If the communication request requires physical interaction, sending the communication request to someone who will not be able to execute the communication request will be fruitless and only serve to delay the communication request and to increase the bandwidth in the system unnecessarily. To overcome such problems the message handler may be arranged to also select a recipient (layer) based on the distance of a recipient to the sender.

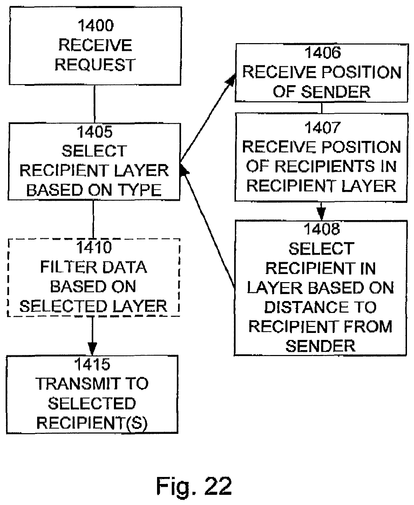

Figure 22 shows a flow chart of how the general method according to herein is expanded by the added functionality of selecting a recipient in a recipient layer based on the distance to the sender. The message handler receives the position 1406 of the sending mobile communication terminal. The position maybe received as part of the message or from a position determining device or by prompting the sending mobile communication terminal. The message handler also receives the position 1407 of potentially receiving mobile communication terminals in the selected recipient layers). The position maybe received as part of the message or from a position determining device or by prompting the potentially receiving mobile communication terminal. At least one recipient in the recipient layer is then selected as recipient for the message or communication request.

If no recipient in the selected recipient layer is close enough a new recipient layer may be selected to ensure that the communication request can be handled by a recipient.

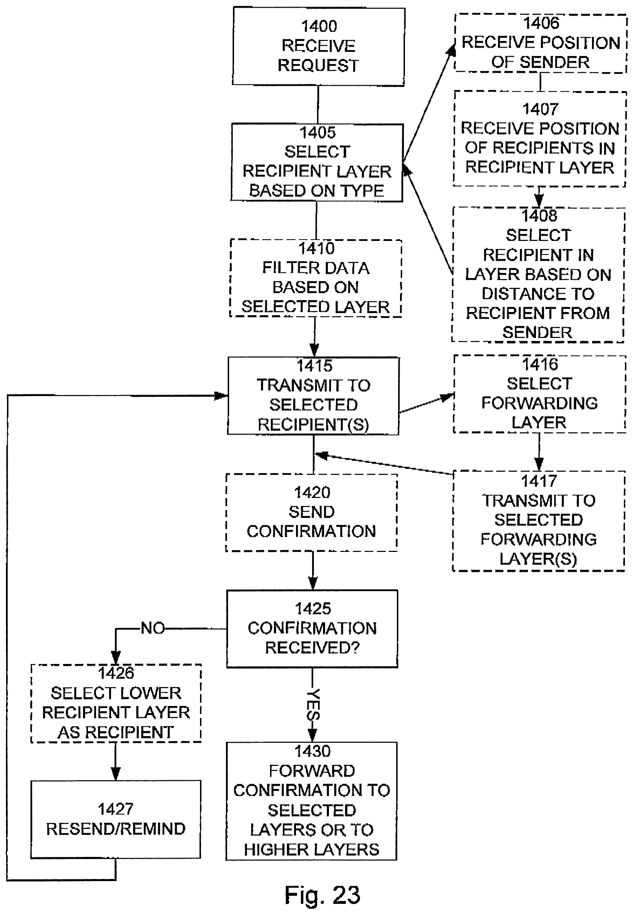

Figure 23 shows a flow chart of a general method according to herein encompassing all features disclosed in the above, wherein some features are indicated to be optional.

### EXEMPLARY EMBODIMENT

The teachings herein may for example be implemented in a telecare communication system for enabling a smoother communication between a person who is to be cared for, such as a senior, and those caring for the person cared for. As is known, many different persons may be involved in caring for a senior, such as immediate family, doctors, care givers, neighbours and volunteers to name a few. Of these many have different obligations and also attachments to the person cared for and will thus only be interested in or involved in certain types of communications. Also, not all persons involved should have access to all data that may be transmitted.

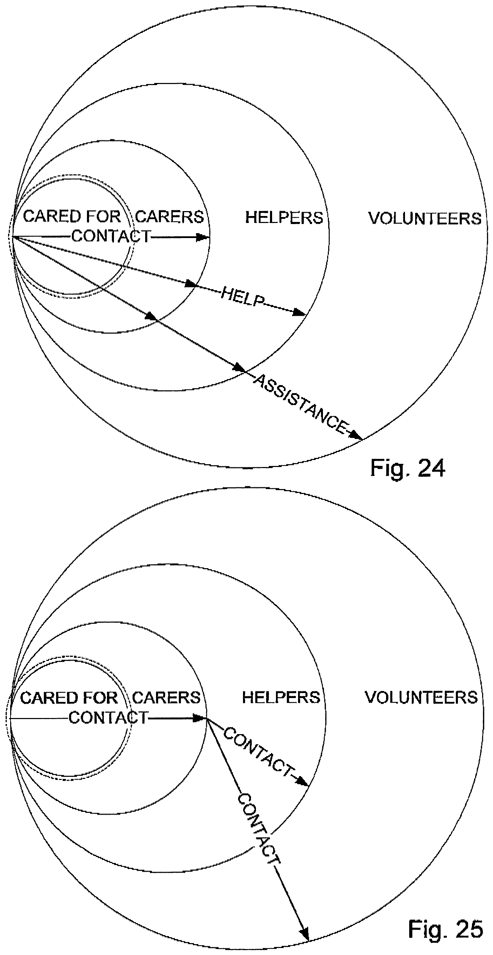

Figure 24 shows a schematic overview of how a hierarchical arrangement such as disclosed in the above may be used for a telecare communication system. In this telecare communication system the SENDER is represented by the person cared for and is referenced CARED FOR.

The highest recipient layer RECIPIENT LAYER 1 is referenced CARERS and includes person(s) with close relation to the senior - in most cases relatives.

The middle recipient layer RECIPIENT LAYER 2 is referenced HELPERS and includes a (closed) network of friends often living close to the senior that agree to help when needed but without any direct responsibility to support. And the lowest recipient layer RECIPIENT LAYER 3 is referenced VOLUNTEERS and includes an (open) network of persons signed up to support any people in need of help when possible.

The different types of communication requests or message are, but are not limited to, a CONTACT request, a HELP request and an ASSISTANCE request. As has been indicated, other types of communication requests and messages are also possible, and will also be disclosed below.

A CONTACT request is a communication request for the closest layer (the CARER) to get in contact without the feeling of disturbing the "busy life" of the carer.

A HELP request is a communication request for the closest layer (the CARER) and the middle layer (the HELPER) to get some support, for example helping with a day to day life problem, such as closing a window or do some shopping. The HELP request may comprise data on what problem needs to be solved. Different data for different recipient layers may consist of sensitive information such as door codes or PIN numbers for credit cards.

An ASSISTANCE request is a communication request for assistance being an emergency request, and may be initiated for example through the press of a dedicated key on the mobile communication terminal. The emergency request is sent to CARERs and HELPERs and may also be sent to VOLUNTEERS if so specified in the message handler. The request may comprise a position of the cared for person and also an indication of the type of emergency and if any medicines or special competences are required.

One benefit of applying a communication system hierarchy as described herein to a telecare communication system is that the cared for person does not need to worry about who a communication request is sent to. As cared for person does not know who he is contacting, he may not be held back by fearing to be of nuisance. This is an important aspect for seniors in many modem cultures and as it is easier to place an anonymous request than a request to a specific person, the senior may utilize the telecare communication system more often and thus improve his quality of life. Furthermore, the system herein also enables the carer to receive updates on what requests are being made and if they are being handled and will thereby be at ease knowing that their loved one is cared for. Also, the telecare communication system as herein simplifies daily contact between a person cared for and the persons caring or supporting the person cared for through convenient communication, notification and request functions.

Another benefit is that a telecare communication system as herein helps protecting the integrity of a person cared for as data and other sensitive information is kept secret to certain recipients only and also as the telecare communication system allows for a protected identity of the recipients.

Figure 25 shows a schematic overview of how a hierarchical arrangement such as disclosed in the above may be used for forwarding a request, wherein a CONTACT request is received by a CARER and forwarded to the HELPER recipient layer and/or the VOLUNTEER recipient layer.

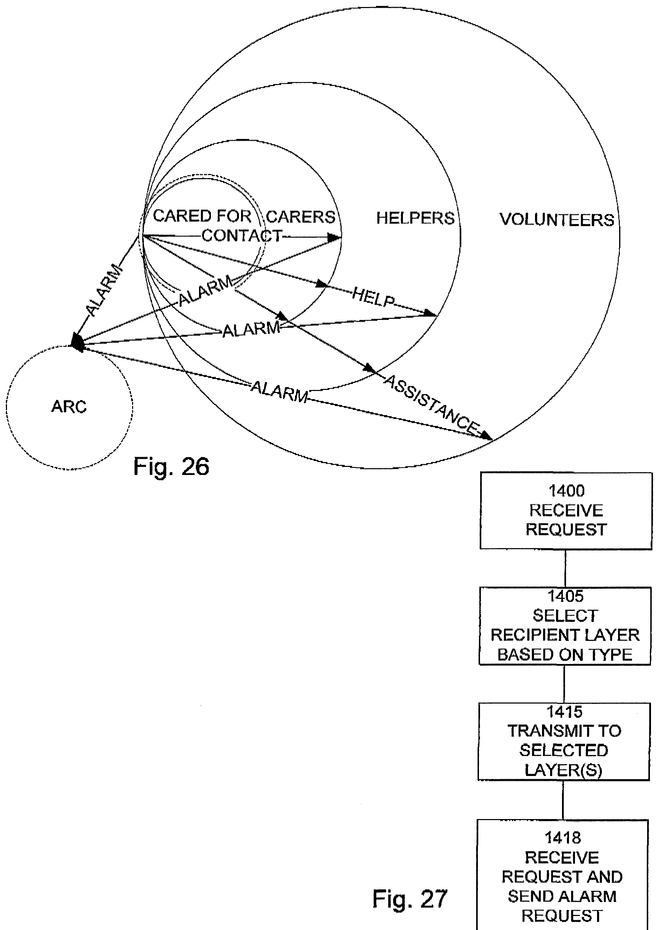

One recipient layer or a specific recipient may be an alarm service provider, such as an alarm center, that a recipient (or the sender) may choose to contact for further assistance. In figure 26, showing a schematic overview of a telecare communication system hierarchy including an alarm center, such as an alarm receiving center ARC which has been indicated to be outside the recipient layers, but it should be noted that the alarm center may be one recipient layer. Alarm requests may be initiated by both the sender and/or any recipient. An alarm request may be initiated at will or as a response to receiving a request as indicated in figure 26.

As has been disclosed for the assistance request, an alarm request may also be initiated from a dedicated key on the mobile communication terminal. However, an alarm request may also be generated through a press on a key in a separate alarm device, such as a panic button or an alarm accessory.

Figure 27 shows a flowchart of how a general method according to herein may be expanded to include this alarm functionality. As a communication request has been transmitted and received an alarm request is generated and transmitted 1418 to an alarm center.

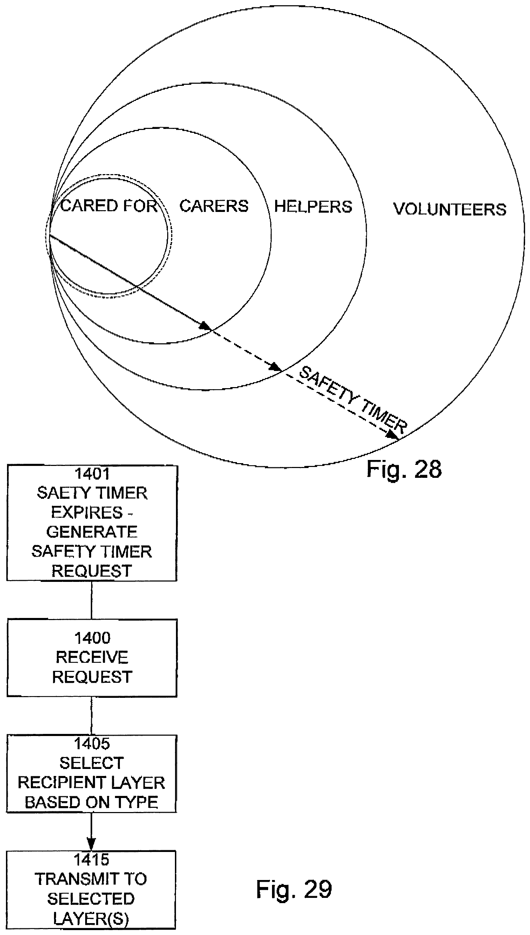

To allow for a closer monitoring of a person cared for, the telecare communication system may be equipped with a safety timer functionality. A safety timer is set, either by the person cared for or a recipient such as a carer, or other administrator, possibly even the alarm center, and as the safety timer expires without any actions have been taken by the person cared for a safety timer request SAFETY TIMER is transmitted to one or more of the recipient layers. An action that could be taken by the cared for person is to cancel or respond to a prompt that is generated and provided (such as displaying a pop up on the display of the mobile communication terminal). Figure 28 shows a schematic overview of a telecare communication system hierarchy including a safety timer functionality and figure 29 shows a flowchart of how a general method according to herein may be expanded to include this safety timer functionality. As a safety timer is set and expires a safety timer request is generated 1405 and the safety timer request is received 1400 by the message handler.

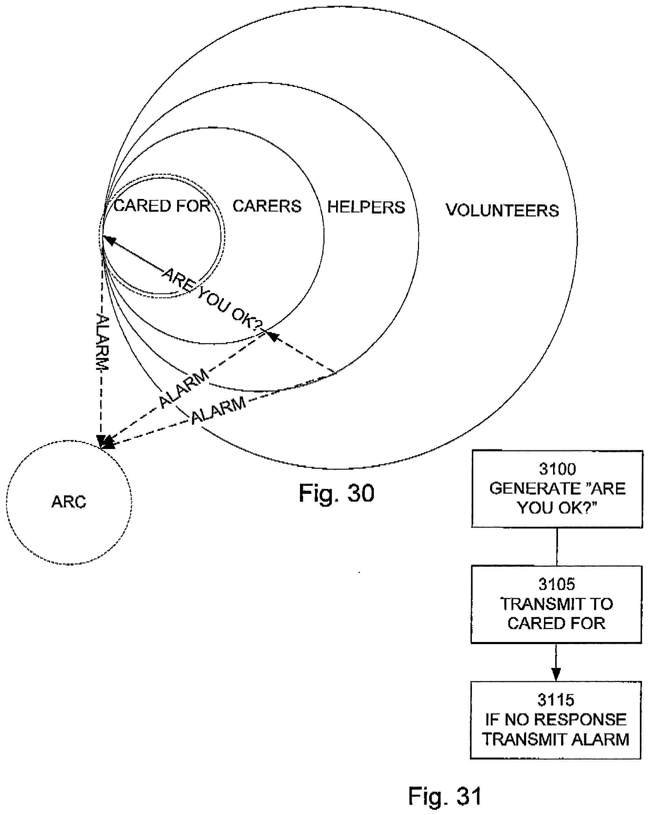

To also enable for a closer monitoring of a person cared for, the telecare communication system may be equipped with a functionality for prompting a person cared for to determine whether the person cared for is experiencing or having any problems. A communication request requiring a response, an are you OK (ARE YOU OK?) request, is generated and transmitted by a recipient, such as a carer or a helper, or even an alarm center or a volunteer. Such a request may be sent sporadically (manually) or regularly (automatically). If no response is received, possibly within a timeout period, an alarm request may be generated and transmitted, possibly to the alarm center. Figure 30 shows a schematic overview of a telecare communication system hierarchy including such are you ok functionality and figure 31 shows a flowchart of this prompting functionality. A prompting request (ARE YOU OK) is generated 3100 and transmitted to the person cared for 3105. If no response is received back from the person cared for an alarm request is generated and transmitted 3115.

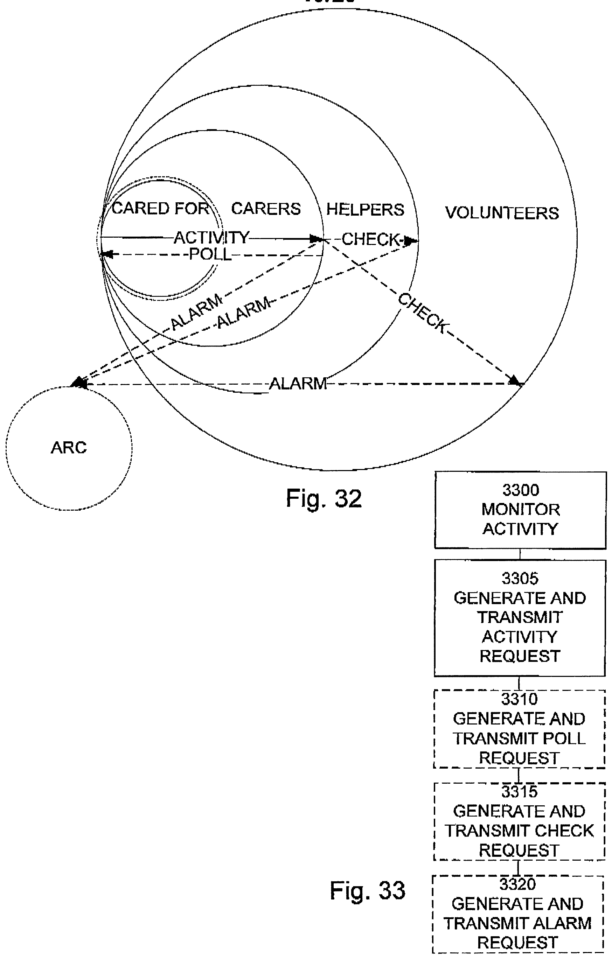

A similar function is also enabled by a telecare communication system according to herein, namely to monitor the activity of a person cared for and if no activity is detected - or a specific activity is detected - an ACTIVITY message is generated and transmitted to a carer. The carer may then generate a request for an explanation or to check a status of the cared for person, such request asks for further information and is referenced POLL in figure 32 which shows a schematic overview of a telecare communication system hierarchy including such monitoring functionality. In one embodiment the POLL request is an ARE YOU OK request As is also shown in figure 32, the carer may also generate and transmit requests asking a helper or a volunteer to check (a CHECK request) on the person cared for. If a carer or helper or volunteer determine that further assistance is required an alarm request may be generated and transmitted to an alarm center. In one embodiment the polling request is generated and transmitted automatically if no activity is detected or if a specific activity is detected as a response to the activity message. In one embodiment an alarm request is generated automatically in response to an ACTIVITY message for a specific activity, possibly such a demand for an alarm request is specified in the activity message.

In one embodiment the CHECK request may be generated automatically in response to determining that no response to a POLL request has been received, possibly within, a time period.

One activity that can be monitored is the placement and acceptance of voice or video calls and if no voice calls or video calls are detected within a time period, an ACTIVITY request is generated. One activity that can be monitored is the sending and opening of messages and if no messages are detected within a time period, an ACTIVITY request is generated. One activity that can be monitored is the use of an application such as a browsing application and if an application is not used within a time period, an ACTIVITY request is generated. One activity that can be monitored is a movement of the mobile communication terminal of the person cared for and if no movement is detected within a time period, an ACTIVITY request is generated. One activity that can be monitored the network status of the mobile communication terminal of the person cared for, that is if the person is online or not, and if it is detected that a cared for person's mobile communication terminal is offline for a time period, an ACTIVITY request is generated. It should be noted that a combination of the activities may also be monitored within a same time period. The ACTIVITY request may comprise information on what activity has been monitored.

Figure 33 shows a flowchart of this monitoring functionality where an activity is monitored and based on such determination an ACTIVITY request is generated and transmitted 3305. This may be done as in the flowchart of figure 23. Optionally the receiving carer may generate and transmit a POLL request to the person cared for 3310. Also optionally the receiving carer may generate and transmit a CHECK request to a helper or a volunteer 3315.

If no responses are received within a time period, an ALARM REQUEST may be generated and transmitted to an alarm center 3320.

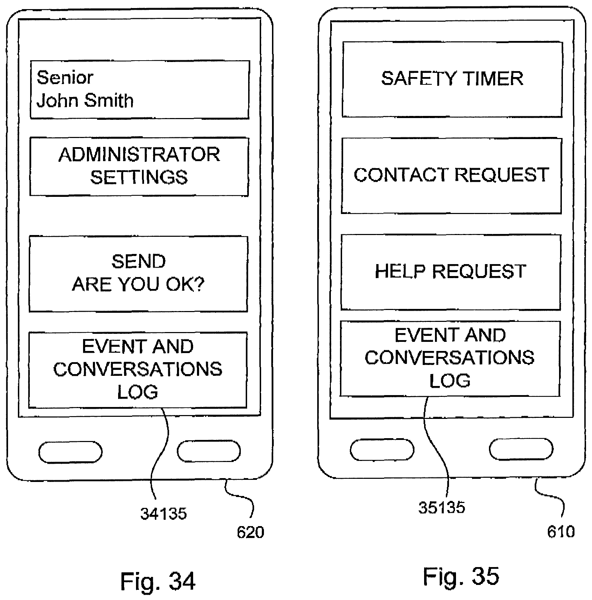

Figure 34 shows an overview of a mobile communication terminal 620 (also referring to figure 6) to be used by a carer. The mobile communication terminal 620 is configured to display a graphical user interface comprising four virtual keys 34135. One virtual key is for accessing administrator settings, and one virtual key is for displaying information about the person cared for. Also shown are virtual keys for sending an "Are you ok?" request (SEND ARE YOU OK?)and a virtual key for accessing a log of events and conversations (EVENT AND CONVERSATIONS LOG), possibly including requests and confirmations, relating to the person cared for.

Figure 35 shows an overview of a mobile communication terminal 610 (also referring to figure 6) to be used by a person cared for. The mobile communication terminal 610 is configured to display a graphical user interface comprising at least one virtual key 35135. The virtual key(s) is/are arranged to generate and transmit a request when they are activated or pressed. The type of the request is determined based on which virtual key 34135 is pressed. Alternatively, the mobile communication terminal 610 may also comprise physical keys (referenced 130 in figure 1) being associated with generating a request of a specific type. As a controller of the mobile communication terminal 610 receives an actuation of the key, the message or request is generated and associated with the message type being associated with the activated key. In the example of figure 35 four virtual keys are displayed 35135. One being for initiating a safety timer, and as it expires generate and transmit a safety timer request as has been disclosed above in relation to figures 28 and 29; one being for generating and sending a contact request (REQUEST); one being for generating and transmitting a help request (HELP REQUEST) and one being for accessing a log of events and conversations possibly including requests and confirmations (EVENT AND CONVERSATIONS LOG).

The teachings herein have mainly been described above with reference to a few embodiments. However, as is readily appreciated by a person skilled in the art, other embodiments than the ones disclosed above are equally possible within the scope of the invention, as defined by the appended patent claims.

## Claims

1. A communication apparatus (100, 200, 300, 400, 610, 650) comprising a controller (210), wherein said controller (210) is configured to:
receive a message in the form of a communication request;
determine a type for said message;
in a recipient layer hierarchy in which a higher recipient layer is a layer with recipients having a higher priority or clearance or a stronger association with a message sender, and a lower recipient layer is a layer with recipients having a lower priority or clearance or a weaker if any association with the message sender, select a first recipient layer based on said type, said first recipient layer comprising at least two recipients;
transmit said message to at least one of said at least two recipients of said first recipient layer;
receive a confirmation from said at least one of said at least two recipients of said first recipient layer; and in response thereto
transmit said confirmation received from said at least one of said at least two recipients of said first recipient layer to at least another one of said at least two recipients of said first recipient layer,
wherein said controller (210) is further configured to:
select a second recipient layer, said second recipient layer comprising at least one recipient and being a higher recipient layer than said first recipient layer, and to
transmit said confirmation from said at least one of said at least two recipients of said first recipient layer to at least one of said at least one recipient of said second recipient layer,
and wherein recipients of a highest recipient layer are informed of the status or progress of all communication requests by receiving every communication request and also receiving every confirmation.

2. The communication apparatus (100, 200, 300, 400, 610, 650) according to claim 1, wherein said controller (210) is further configured to
determine that no confirmation has been received, and in response thereto
retransmit said message to said at least one of said at least two recipients of said first recipient layer; and/or transmit a reminder of said message to said at least one of said at least one recipient of said first recipient layer.

3. The communication apparatus (100, 200, 300, 400, 610, 650) according to claim 2, wherein said controller (210) is further configured to select a third recipient layer, said third recipient layer comprising at least one recipient and being a lower recipient layer than said first recipient layer, and to retransmit said message to at least one of said at least one recipient of said third recipient layer; and/or transmit a reminder of said message to at least one of said at least one recipient of said third recipient layer.

4. The communication apparatus (100, 200, 300, 400, 610, 650) according to any preceding claim, wherein said message comprises data and said controller (210) is further configured to filter said data based on said message type before transmitting said message to at least one of said at least two recipients of said first recipient layer.

5. The communication apparatus (100, 200, 300, 400, 610, 650) according to claim 3, wherein said controller (210) is further configured to receive a selected forwarding layer, being a fourth recipient layer comprising at least one recipient and transmit said message to at least one of said at least one recipient of said fourth recipient layer.

6. The communication apparatus (100, 200, 300, 400, 610, 650) according to any preceding claim, wherein said controller (210) is further configured to:
identify a sender of said message;
receive a position of said sender;
receive at least one position of at least one recipient of said first recipient layer;
determine a distance between said sender and said at least one recipient of said first recipient layer; and
based on said distance, select said at least one of said at least one recipient of said first recipient layer to transmit said message to.

7. A method for use in a communication apparatus (100, 200, 300, 400, 610, 650), wherein said method comprises
receiving a message in the form of a communication request;
determining a type for said message;
in a recipient layer hierarchy in which a higher recipient layer is a layer having recipients with a higher priority or clearance or a stronger association with a message sender, and a lower recipient layer is a layer with recipients having a lower priority or clearance or a weaker if any association with the message sender, selecting a first recipient layer based on said type, said first recipient layer comprising at least two recipients; transmitting said message to at least one of said at least two recipients of said first recipient layer;
receiving a confirmation from said at least one of said at least two recipients of said first recipient layer; in response thereto
transmitting said received confirmation from said at least one of said at least two recipients of said first recipient layer to at least another one of said at least two recipients of said first recipient layer;
selecting a second recipient layer, said second recipient layer comprising at least one recipient and being a higher recipient layer than said first recipient layer; and
transmitting said confirmation from said at least one of said at least two recipients of said first recipient layer to at least one of said at least one recipient of said second recipient layer,
wherein recipients of a highest recipient layer are informed of the status or progress of all communication requests by receiving every communication request and also receiving every confirmation.

8. A communications system (600) comprising a communication apparatus (100, 200,300,400,610, 650) according to claim 1, a first mobile communication terminal (100, 200, 610) and a second mobile communication terminal (100, 200, 620), wherein said first mobile communication terminal (100, 200, 610) is configured to generate a message in the form of a communication request, and associate said message with a type, and wherein said second mobile communication terminal (100, 200, 620) is associated with said first recipient layer and configured to receive said message.

9. The communications system (600) according to claim 8, wherein said first mobile communication terminal (100, 200, 610) is further configured to determine that a safety timer has expired and in response thereto generate said message and associate said message with a type being a safety timer request.

10. The communications system (600) according to any of claims 8 to 9, wherein said first mobile communication terminal (100, 200, 610) is further configured to
monitor an activity;
generate said message as an activity request based on said monitored activity.

11. The communications system (600) according to claim 10, wherein said activity relates to one or more of an activity taken from a group comprising: placement and acceptance of voice or video calls; sending and opening of messages; use of an application; movement of a mobile communication terminal belonging to a person cared for; network status of the mobile communication terminal belonging to the person cared for.

12. The communications system (600) according to any of claims 8 to 11, further comprising a key (130, 135, 35135), wherein said first mobile communication terminal (100, 200, 610) is further configured to:
associate said key (130, 135, 35135) with a message type; and
receive an actuation of said key (130, 135, 35135) and in response thereto generate said message, wherein the type of said generated message is the message type associated with the key (130, 135, 35135).

13. A computer-readable storage medium (70) encoded with instructions (71) that, when executed on a processor, perform the method according to claim 7.

## Patentansprüche

1. Kommunikationsvorrichtung (100, 200, 300, 400, 610, 650) mit einer Steuereinrichtung (210), wobei die Steuereinrichtung (210) so konfiguriert ist, dass sie:
eine Nachricht in Form einer Kommunikationsanfrage empfängt,
einen Typ für diese Nachricht bestimmt,
in einer Empfängerschichthierarchie, bei der es sich bei einer höher gestellten Empfängerschicht um eine Schicht mit Empfängern mit einer höheren Priorität oder Freigabe oder einer stärkeren Verbundenheit mit einem Nachrichtenabsender und bei einer niedriger gestellten Empfängerschicht um eine Schicht mit Empfängern mit einer geringeren Priorität oder Freigabe oder einer schwächeren oder keiner Verbundenheit mit dem Nachrichtenabsender handelt, auf der Grundlage des Typs eine erste Empfängerschicht auswählt, wobei die erste Empfängerschicht mindestens zwei Empfänger umfasst,
die Nachricht zu mindestens einem der mindestens zwei Empfänger der ersten Empfängerschicht überträgt,
eine Bestätigung von dem mindestens einen der mindestens zwei Empfänger der ersten Empfängerschicht empfängt und als Reaktion darauf
die von dem mindestens einen der mindestens zwei Empfänger der ersten Empfängerschicht empfangene Bestätigung zu mindestens einem weiteren der mindestens zwei Empfänger der ersten Empfängerschicht überträgt,
wobei die Steuereinrichtung (210) ferner so konfiguriert ist, dass sie:
eine zweite Empfängerschicht auswählt, wobei die zweite Empfängerschicht mindestens einen Empfänger umfasst und es sich bei ihr um eine höher gestellte Empfängerschicht als die erste Empfängerschicht handelt, und dass sie
die Bestätigung von dem mindestens einen der mindestens zwei Empfänger der ersten Empfängerschicht zu mindestens einem des mindestens einen Empfängers der zweiten Empfängerschicht überträgt,
und wobei Empfänger einer höchst gestellten Empfängerschicht über den Status oder den Fortschritt aller Kommunikationsanfragen informiert werden, indem sie jede Kommunikationsanfrage sowie jede Bestätigung empfangen.

2. Kommunikationsvorrichtung (100, 200, 300, 400, 610, 650) nach Anspruch 1, wobei die Steuereinrichtung (210) ferner so konfiguriert ist, dass sie:
bestimmt, dass keine Bestätigung empfangen wurde, und als Reaktion darauf
die Nachricht erneut zu dem mindestens einen der mindestens zwei Empfänger der ersten Empfängerschicht und/oder eine Erinnerung an die Nachricht zu dem mindestens einen des mindestens einen Empfängers der ersten Empfängerschicht überträgt.

3. Kommunikationsvorrichtung (100, 200, 300, 400, 610, 650) nach Anspruch 2, wobei die Steuereinrichtung (210) ferner so konfiguriert ist, dass sie:
eine dritte Empfängerschicht auswählt, wobei die dritte Empfängerschicht mindestens einen Empfänger umfasst und es sich bei ihr um eine niedriger gestellte Empfängerschicht als die erste Empfängerschicht handelt, und
die Nachricht erneut zu mindestens einem des mindestens einen Empfängers der dritten Empfängerschicht und/oder eine Erinnerung an die Nachricht zu mindestens einem des mindestens einen Empfängers der dritten Empfängerschicht überträgt.

4. Kommunikationsvorrichtung (100, 200, 300, 400, 610, 650) nach einem vorhergehenden Anspruch,
wobei die Nachricht Daten umfasst und die Steuereinrichtung (210) ferner so konfiguriert ist, dass sie die Daten auf der Grundlage des Nachrichtentyps filtert, bevor sie die Nachricht zu mindestens einem der mindestens zwei Empfänger der ersten Empfängerschicht überträgt.

5. Kommunikationsvorrichtung (100, 200, 300, 400, 610, 650) nach Anspruch 3, wobei die Steuereinrichtung (210) ferner so konfiguriert ist, dass sie eine ausgewählte Weiterleitungsschicht empfängt, bei der es sich um eine vierte Empfängerschicht handelt, die mindestens einen Empfänger umfasst, und die Nachricht zu mindestens einem des mindestens einen Empfängers der vierten Empfängerschicht überträgt.

6. Kommunikationsvorrichtung (100, 200, 300, 400, 610, 650) nach einem vorhergehenden Anspruch, wobei die Steuereinrichtung (210) ferner so konfiguriert ist, dass sie:
einen Absender der Nachricht identifiziert,
einen Standort des Absenders empfängt,
mindestens einen Standort mindestens eines Empfängers der ersten Empfängerschicht empfängt,
eine Entfernung zwischen dem Absender und dem mindestens einen Empfänger der ersten Empfängerschicht bestimmt und
auf der Grundlage der Entfernung den mindestens einen des mindestens einen Empfängers der ersten Empfängerschicht auswählt, zu dem die Nachricht übertragen werden soll.

7. Verfahren zur Verwendung in einer Kommunikationsvorrichtung (100, 200, 300, 400, 610, 650), wobei das Verfahren Folgendes umfasst:
Empfangen einer Nachricht in Form einer Kommunikationsanfrage,
Bestimmen eines Typs für die Nachricht,
Auswählen einer ersten Empfängerschicht auf der Grundlage des Typs, wobei die erste Empfängerschicht mindestens zwei Empfänger umfasst, in einer Empfängerschichthierarchie, bei der es sich bei einer höher gestellten Empfängerschicht um eine Schicht mit Empfängern mit einer höheren Priorität oder Freigabe oder einer stärkeren Verbundenheit mit einem Nachrichtenabsender und bei einer niedriger gestellten Empfängerschicht um eine Schicht mit Empfängern mit einer geringeren Priorität oder Freigabe oder einer schwächeren oder keiner Verbundenheit mit dem Nachrichtenabsender handelt,
Übertragen der Nachricht zu mindestens einem der mindestens zwei Empfänger der ersten Empfängerschicht, Empfangen einer Bestätigung von dem mindestens einen der mindestens zwei Empfänger der ersten Empfängerschicht, als Reaktion darauf
Übertragen der von dem mindestens einen der mindestens zwei Empfänger der ersten Empfängerschicht empfangenen Bestätigung zu mindestens einem weiteren der mindestens zwei Empfänger der ersten Empfängerschicht,
Auswählen einer zweiten Empfängerschicht, wobei die zweite Empfängerschicht mindestens einen Empfänger umfasst und es sich bei ihr um eine höher gestellte Empfängerschicht als die erste Empfängerschicht handelt, und
Übertragen der Bestätigung von dem mindestens einen der mindestens zwei Empfänger der ersten Empfängerschicht zu mindestens einem des mindestens einen Empfängers der zweiten Empfängerschicht,
wobei Empfänger einer höchst gestellten Empfängerschicht über den Status oder den Fortschritt aller Kommunikationsanfragen informiert werden, indem sie jede Kommunikationsanfrage sowie jede Bestätigung empfangen.

8. Kommunikationssystem (600), das eine Kommunikationsvorrichtung (100, 200, 300, 400, 610, 650) nach Anspruch 1, ein erstes mobiles Kommunikationsendgerät (100, 200, 610) und ein zweites mobiles Kommunikationsendgerät (100, 200, 620) umfasst, wobei das erste mobile Kommunikationsendgerät (100, 200, 610) so konfiguriert ist, dass es eine Nachricht in Form einer Kommunikationsanfrage erzeugt und die Nachricht mit einem Typ verknüpft,
und wobei das zweite mobile Kommunikationsendgerät (100, 200, 620) mit der ersten Empfängerschicht verbunden und so konfiguriert ist, dass es die Nachricht empfängt.

9. Kommunikationssystem (600) nach Anspruch 8, wobei das erste mobile Kommunikationsendgerät (100, 200, 610) ferner so konfiguriert ist, dass es bestimmt, dass ein Sicherheitstimer abgelaufen ist, und als Reaktion darauf die Nachricht erzeugt und die Nachricht mit einem Typ verknüpft, bei dem es sich um eine Sicherheitstimeranfrage handelt.

10. Kommunikationssystem (600) nach einem der Ansprüche 8 und 9, wobei das erste mobile Kommunikationsendgerät (100, 200, 610) ferner so konfiguriert ist, dass es
eine Aktivität überwacht,
auf der Grundlage der überwachten Aktivität die Nachricht als Aktivitätsanfrage erzeugt.

11. Kommunikationssystem (600) nach Anspruch 10, wobei die Aktivität eine oder mehrere Aktivitäten aus einer Gruppe betrifft, die Folgendes umfasst: Tätigen und Annehmen von Sprach- oder Videoanrufen, Senden und Öffnen von Nachrichten, Benutzen einer Anwendung, Bewegen eines mobilen Kommunikationsendgeräts, das einer pflegebedürftigen Person gehört, Netzwerkstatus des mobilen Kommunikationsendgeräts, das der pflegebedürftigen Person gehört.

12. Kommunikationssystem (600) nach einem der Ansprüche 8 bis 11, das ferner eine Taste (130, 135, 35135) umfasst, wobei das erste mobile Kommunikationsendgerät (100, 200, 610) ferner so konfiguriert ist, dass es:
die Taste (130, 135, 35135) mit einem Nachrichtentyp verknüpft und
eine Betätigung der Taste (130, 135, 35135) empfängt und als Reaktion darauf die Nachricht erzeugt, wobei es sich beim Typ der erzeugten Nachricht um den Nachrichtentyp handelt, der mit der Taste (130, 135, 35135) verknüpft ist.

13. Computerlesbares Speichermedium (70), das mit Anweisungen (71) codiert ist, die bei Ausführen auf einem Prozessor das Verfahren nach Anspruch 7 durchführen.

## Revendications

1. Appareil de communication (100, 200, 300, 400, 610, 650) comprenant un contrôleur (210), dans lequel ledit contrôleur (210) est configuré de manière à :
recevoir un message sous la forme d'une demande de communication ;
déterminer un type pour ledit message ;
dans une hiérarchie de couches de destinataires dans laquelle une couche de destinataires supérieure correspond à une couche dont les destinataires présentent une priorité ou une autorisation supérieure, ou une association plus forte avec un expéditeur de message, et une couche de destinataires inférieure correspond à une couche dont les destinataires présentent une priorité ou une autorisation inférieure, ou une association plus faible, le cas échéant, avec l'expéditeur de message, sélectionner une première couche de destinataires sur la base dudit type, ladite première couche de destinataires comprenant au moins deux destinataires ;
transmettre ledit message à au moins un destinataire parmi lesdits au moins deux destinataires de ladite première couche de destinataires ;
recevoir une confirmation en provenance dudit au moins un destinataire parmi lesdits au moins deux destinataires de ladite première couche de destinataires ; et, en réponse à cela
transmettre ladite confirmation reçue en provenance dudit au moins un destinataire parmi lesdits au moins deux destinataires de ladite première couche de destinataires, à au moins un autre destinataire parmi lesdits au moins deux destinataires de ladite première couche de destinataires ;
dans lequel ledit contrôleur (210) est en outre configuré de manière à :
sélectionner une deuxième couche de destinataires, ladite deuxième couche de destinataires comprenant au moins un destinataire et correspondant à une couche de destinataires plus élevée que ladite première couche de destinataires ; et
transmettre ladite confirmation, dudit au moins un destinataire parmi lesdits au moins deux destinataires de ladite première couche de destinataires à au moins un destinataire dudit au moins un destinataire de ladite deuxième couche de destinataires ; et
dans lequel des destinataires d'une couche de destinataires la plus élevée sont tenus informés du statut ou de l'avancement de toutes les demandes de communication en recevant chaque demande de communication et également en recevant chaque confirmation.

2. Appareil de communication (100, 200, 300, 400, 610, 650) selon la revendication 1, dans lequel ledit contrôleur (210) est en outre configuré de manière à :
déterminer qu'aucune confirmation n'a été reçue, et en réponse à cela,
retransmettre ledit message audit au moins un destinataire parmi lesdits au moins deux destinataires de ladite première couche de destinataires ; et/ou transmettre un rappel dudit message audit au moins un destinataire dudit au moins un destinataire de ladite première couche de destinataires.

3. Appareil de communication (100, 200, 300, 400, 610, 650) selon la revendication 2, dans lequel ledit contrôleur (210) est en outre configuré de manière à sélectionner une troisième couche de destinataires, ladite troisième couche de destinataires comprenant au moins un destinataire et correspondant à une couche de destinataires inférieure à ladite première couche de destinataires, et à retransmettre ledit message à au moins un destinataire dudit au moins un destinataire de ladite troisième couche de destinataires ; et/ou transmettre un rappel dudit message à au moins un destinataire dudit au moins un destinataire de ladite troisième couche de destinataires.

4. Appareil de communication (100, 200, 300, 400, 610, 650) selon l'une quelconque des revendications précédentes, dans lequel ledit message comprend des données, et dans lequel ledit contrôleur (210) est en outre configuré de manière à filtrer lesdites données sur la base dudit type de message avant de transmettre ledit message à au moins un destinataire parmi lesdits au moins deux destinataires de ladite première couche de destinataires.

5. Appareil de communication (100, 200, 300, 400, 610, 650) selon la revendication 3, dans lequel ledit contrôleur (210) est en outre configuré de manière à recevoir une couche d'acheminement sélectionnée, laquelle correspond à une quatrième couche de destinataires comprenant au moins un destinataire, et à transmettre ledit message à au moins un destinataire dudit au moins un destinataire de ladite quatrième couche de destinataires.

6. Appareil de communication (100, 200, 300, 400, 610, 650) selon l'une quelconque des revendications précédentes, dans lequel ledit contrôleur (210) est en outre configuré de manière à :
identifier un expéditeur dudit message ;
recevoir une position dudit expéditeur ;
recevoir au moins une position d'au moins un destinataire de ladite première couche de destinataires ;
déterminer une distance entre ledit expéditeur et ledit au moins un destinataire de ladite première couche de destinataires ; et
sur la base de ladite distance, sélectionner ledit au moins un destinataire dudit au moins un destinataire de ladite première couche de destinataires auquel transmettre ledit message.

7. Procédé destiné à être utilisé dans un appareil de communication (100, 200, 300, 400, 610, 650), dans lequel ledit procédé comprend les étapes ci-dessous consistant à :
recevoir un message sous la forme d'une demande de communication ;
déterminer un type pour ledit message ;
dans une hiérarchie de couches de destinataires dans laquelle une couche de destinataires supérieure correspond à une couche dont les destinataires présentent une priorité ou une autorisation supérieure, ou une association plus forte avec un expéditeur de message, et une couche de destinataires inférieure correspond à une couche dont les destinataires présentent une priorité ou une autorisation inférieure, ou une association plus faible, le cas échéant, avec l'expéditeur de message, sélectionner une première couche de destinataires sur la base dudit type, ladite première couche de destinataires comprenant au moins deux destinataires ;
transmettre ledit message à au moins un destinataire parmi lesdits au moins deux destinataires de ladite première couche de destinataires ;
recevoir une confirmation en provenance dudit au moins un destinataire parmi lesdits au moins deux destinataires de ladite première couche de destinataires ; et, en réponse à cela
transmettre ladite confirmation reçue en provenance dudit au moins un destinataire parmi lesdits au moins deux destinataires de ladite première couche de destinataires, à au moins un autre destinataire parmi lesdits au moins deux destinataires de ladite première couche de destinataires ;
sélectionner une deuxième couche de destinataires, ladite deuxième couche de destinataires comprenant au moins un destinataire et correspondant à une couche de destinataires plus élevée que ladite première couche de destinataires ; et
transmettre ladite confirmation, dudit au moins un destinataire parmi lesdits au moins deux destinataires de ladite première couche de destinataires à au moins un destinataire dudit au moins un destinataire de ladite deuxième couche de destinataires ; et
dans lequel des destinataires d'une couche de destinataires la plus élevée sont tenus informés du statut ou de l'avancement de toutes les demandes de communication en recevant chaque demande de communication et également en recevant chaque confirmation.

8. Système de communication (600) comprenant un appareil de communication (100, 200, 300, 400, 610, 650) selon la revendication 1, un premier terminal de communication mobile (100, 200, 610) et un second terminal de communication mobile (100, 200, 620), dans lequel ledit premier terminal de communication mobile (100, 200, 610) est configuré de manière à générer un message sous la forme d'une demande de communication, et à associer ledit message à un type ; et
dans lequel ledit second terminal de communication mobile (100, 200, 620) est associé à ladite première couche de destinataires et est configuré de manière à recevoir ledit message.

9. Système de communication (600) selon la revendication 8, dans lequel ledit premier terminal de communication mobile (100, 200, 610) est en outre configuré de manière à déterminer qu'une minuterie de sécurité a expiré, et en réponse à cela, à générer ledit message et à associer ledit message à un type correspondant à une demande de minuterie de sécurité.

10. Système de communication (600) selon l'une quelconque des revendications 8 à 9, dans lequel ledit premier terminal de communication mobile (100, 200, 610) est en outre configuré de manière à :
surveiller une activité ; et
générer ledit message sous la forme d'une demande d'activité sur la base de ladite activité surveillée.

11. Système de communication (600) selon la revendication 10, dans lequel ladite activité se rapporte à une ou plusieurs actions d'une activité sélectionnée à partir d'un groupe comprenant : le placement et l'acceptation d'appels vocaux ou vidéo ; l'envoi et l'ouverture de messages ; l'utilisation d'une application ; le déplacement d'un terminal de communication mobile appartenant à une personne prise en charge ; un statut de réseau du terminal de communication mobile appartenant à la personne prise en charge.

12. Système de communication (600) selon l'une quelconque des revendications 8 à 11, comprenant en outre une touche (130, 135, 35135), dans lequel ledit premier terminal de communication mobile (100, 200, 610) est en outre configuré de manière à :
associer ladite touche (130, 135, 35135) à un type de message ; et
recevoir un actionnement de ladite touche (130, 135, 35135), et en réponse à cela, générer ledit message, dans lequel le type dudit message généré est le type de message associé à la touche (130, 135, 35135).

13. Support de stockage lisible par ordinateur (70) codé avec des instructions (71) qui, lorsqu'elles sont exécutées sur un processeur, mettent en oeuvre le procédé selon la revendication 7.
